# EUROPEAN PATENT APPLICATION

(11) **EP 4 501 228 A1**
(43) Date of publication of application: **05.02.2025**
(21) Application number: 23788438.2
(22) Date of filing: 06.01.2023
(51) Int. Cl.: A61B 5/155, A61B 5/15, A61B 5/145

(54) **APPLICATOR AND APPLICATOR ASSEMBLY**

(30) Priority: 12.04.2022 KR 20220045053
(71) Applicant: i-Sens, Inc., Seoul 06646 (KR)
(72) Inventor: CHOI, Won Seok, Seoul 06646 (KR); CHOI, Hyun Ho, Seoul 06646 (KR)
(74) Representative: BCKIP Part mbB
(86) International application number: PCT/KR2023/000321
(87) International publication number: WO 2023/200078

(57) **Abstract**

An applicator assembly according to the present disclosure includes a medical device including a sensor unit having a sensor of which at least a portion is to be inserted into skin of a user and a base unit attached to the skin of the user to be coupled to the sensor unit, an applicator body including an applicator body bottom portion disposed to face the skin of the user and an applicator body recess formed to the applicator body bottom portion wherein the base unit is disposed, and an insertion unit disposed to the applicator body for moving the sensor unit from a first position spaced apart from the base unit to a second position for being coupled to the base unit, and the base unit is disposed to the applicator body recess to protrude from the applicator body bottom portion.

## Description

### Technical Field

The present disclosure relates to an applicator. Specifically, the present disclosure relates to an applicator and an applicator assembly for attaching, to skin of a user, a medical device attached to the skin of the user to be used.

### Background Art

With recent development of a medical technology, various medical devices attached to a body of a user are developed and sold. A medical device attached to the body of the user may be attached to skin of a patient with a chronic disease to monitor biometric information or be helpfully used in treatment.

For example, a chronic disease such as diabetes requires continuous care, and a medical device that is attached to skin to measure blood sugar may be used for monitoring blood sugar of a patient with diabetes. Diabetes has a feature that a subjective symptom thereof is not approximately obvious at an initial stage. As diabetes progresses, unique symptoms of diabetes, such as eating much, drinking much, polyuria, weight loss, general malaise, itching, and hand and foot injuries that are not cured and last long, appear. When diabetes further progresses, complications that develop into visual disturbance, high blood pressure, kidney disease, stroke, periodontal disease, a cramp, neuralgia, gangrene, and the like appear. In order to diagnose diabetes and manage diabetes to prevent developing the complications, systematic measurement and treatment of the blood sugar may be done simultaneously.

For the patient with diabetes and people having sugar measured to be more than normal in blood, multiple medical device manufacturers provides various types of blood sugar meters for measuring the blood sugar.

A scheme of a blood sugar meter includes a scheme of gathering blood from a finger tip of the user to perform blood sugar measurement on a one-time basis and a scheme of being attached to a stomach and an arm of the user to continuously perform the blood sugar measurement.

In a case of the patient with diabetes, a hyperglycemia state and a hypoglycemia state generally alternate with each other. An emergency may occur in the hypoglycemia state, and the patient may lose consciousness or may die when the hypoglycemia state lasts long without supply of sugar. Thus, immediate detection of the hypoglycemia state is greatly important for the patient with diabetes. However, accurate identification thereof is limited with a blood gathering-type blood sugar meter that intermittently measures the blood sugar.

Recently, in order to overcome such limitation, a continuous glucose monitoring system (CGMS) that is inserted to measure a value of the blood sugar at intervals of several minutes is developed and used. In order to minimize a pain or an aversion of the user, which is caused by blood gathering, the continuous glucose monitoring system may insert a needle-shaped percutaneous sensor into a region such as a stomach and an arm at which the pain is relatively less and then continuously measure the blood sugar.

The continuous glucose monitoring system comprises a sensor unit including the percutaneous sensor inserted into skin of the user to measure the blood sugar in the body, a transmitter for transmitting the value of the blood sugar which is measured by the percutaneous sensor, a terminal outputting the transmitted value of the blood sugar, and the like.

A medical system using the percutaneous sensor is manufactured in various forms by each manufacturer, and a scheme of use thereof also varies. Currently, most systems are manufactured and distributed in a type that attaches a disposable sensor unit to a body through an applicator. The user is to perform tasks of several operations in order to attach the sensor unit to the skin by using the applicator and is to perform several follow-up procedures such as directly pulling out a needle inserted together with the percutaneous sensor into the skin after attaching the sensor unit to the body.

For example, the user may remove packing of the disposable sensor unit and accurately mount the sensor unit to the applicator and may operate the applicator in a state in which the sensor unit is inserted into the applicator and attach the sensor unit to the skin. In addition, after attaching the sensor unit, there is inconvenience of performing tasks such as pulling out the needle inserted into the skin and coupling the transmitter to the sensor unit.

### Detailed Description of the Invention

### Technical Goals

The present disclosure is to provide an applicator and an applicator assembly that minimize an additional task of a user for attaching a medical device to skin as the medical device may be manufactured in an assembled state and are conveniently used to attach the medical device to the skin.

Also, the present disclosure is to provide an applicator and an applicator assembly that may further stably attach the medical device to the skin of the user.

### Technical solutions

According to the present disclosure, there is provided an applicator assembly including a medical device including a sensor unit having a sensor of which at least a portion is to be inserted into skin of a user and a base unit attached to the skin of the user to be coupled to the sensor unit, an applicator body including an applicator body bottom portion disposed to face the skin of the user and an applicator body recess formed to the applicator body bottom portion wherein the base unit is disposed, and an insertion unit disposed to the applicator body for moving the sensor unit from a first position spaced apart from the base unit to a second position for being coupled to the base unit, and the base unit is disposed to the applicator body recess to protrude from the applicator body bottom portion.

The applicator body may include a plurality of floor ribs disposed around the applicator body recess to protrude from the applicator body bottom portion.

The applicator assembly may include a locking hook that is provided on the applicator body, wherein at least a portion of the locking hook protrudes from the applicator body bottom portion and that is configured to engage with a portion of the base unit, wherein the portion of the base unit protrudes from the applicator body bottom portion.

The sensor may be configured to be in contact with an electrical contact part of the base unit when the sensor unit moves to the second position to be coupled to the base unit, and the medical device may be configured to be activated as the sensor is in contact with the electrical contact part.

A protrusion height to which the base unit protrudes from the applicator body bottom portion is desired to have a size greater than or equal to a half of a thickness of the base unit.

According to the present disclosure, there is also provided an applicator for attaching, to skin of a user, a medical device including a sensor unit having a sensor of which at least a portion is to be inserted into the skin of the user and a base unit attached to the skin of the user to be coupled to the sensor unit, the applicator including an applicator body including an applicator body bottom portion disposed to face the skin of the user and an applicator body recess formed to the applicator body bottom portion wherein the base unit is disposed, and an insertion unit disposed to the applicator body for moving the sensor unit from a first position spaced apart from the base unit to a second position for being coupled to the base unit, and a depth of the applicator body recess is smaller than a thickness of the base unit wherein the base unit is to protrude from the applicator body bottom portion when the base unit is disposed to the applicator body recess.

### Effects of the Invention

According to the present disclosure, it is possible to minimize an additional task of a user for attaching a medical device to skin of the user and attach the medical device to the skin of the user only with simply operating an applicator.

According to the present disclosure, since a base unit is mounted to an applicator body to protrude from the applicator body, the user may insert a sensor into the skin while identifying a position of the base unit which is disposed on the skin.

According to the present disclosure, the base unit which protrudes from the applicator body may press and flatten the skin into which the sensor is to be inserted. Thus, the sensor and a needle may be further stably inserted into the skin.

### Brief Description of Drawings

FIG. 1 is a perspective diagram illustrating an applicator assembly according to an example embodiment of the present disclosure.
FIG. 2 illustrates a shape in which a medical device of an applicator assembly according to an example embodiment of the present disclosure is attached to skin of a user.
FIG. 3 is a cross-sectional diagram illustrating a shape before a sensor unit and a base unit of a medical device being coupled.
FIG. 4 is a cross-sectional diagram illustrating a shape in which a sensor unit and a base unit are coupled.
FIG. 5 illustrates a base unit of a medical device.
FIG. 6 is a cross-sectional diagram taken along line I-I of FIG. 1.
FIG. 7 is a cross-sectional diagram taken along line II-II of FIG. 1.

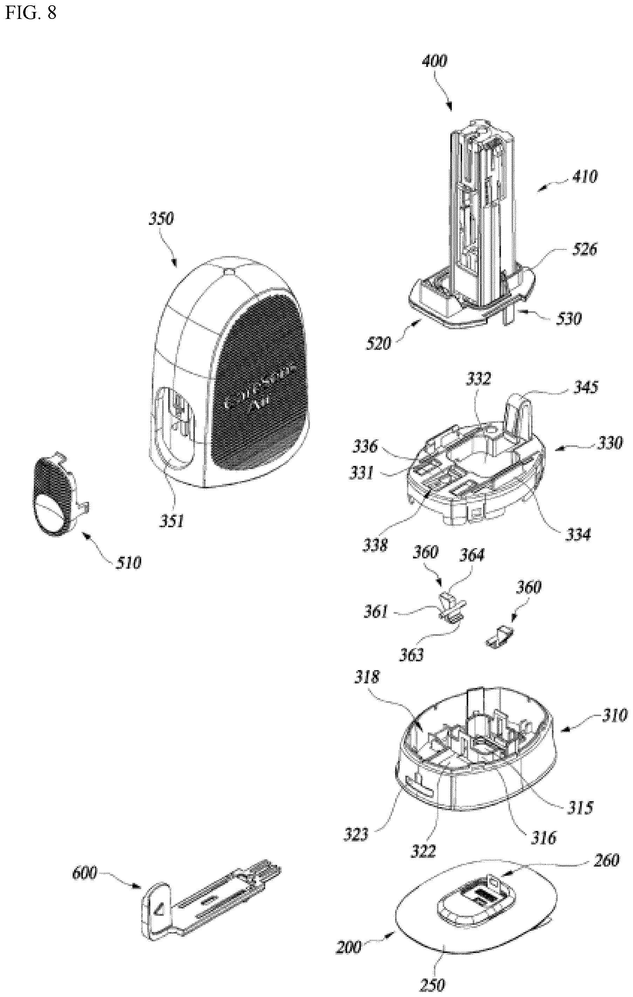

FIG. 8 is a perspective diagram in which an applicator assembly according to an example embodiment of the present disclosure is disassembled and illustrated.
FIG. 9 illustrates a shape in which a base unit of an applicator assembly according to an example embodiment of the present disclosure is coupled to an applicator body.
FIG. 10 illustrates movement of an operation part provided to an applicator assembly according to an example embodiment of the present disclosure.

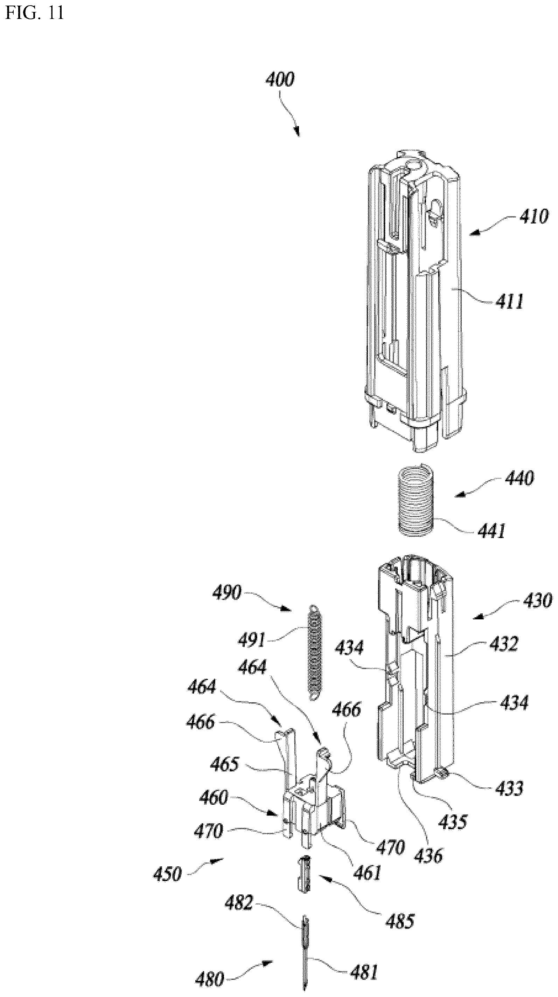

FIG. 11 is an illustration in which an insertion unit assembly of an applicator is disassembled.
FIG. 12 illustrates a moving tab and a protective sheet of an applicator assembly according to an example embodiment of the present disclosure.
FIGS. 13 through 16 illustrate a process of attaching a medical device to skin of a user by using an applicator.

### Mode for Carrying Out the Invention

Hereinafter, an applicator and an applicator assembly will be described in detail with reference to drawings.

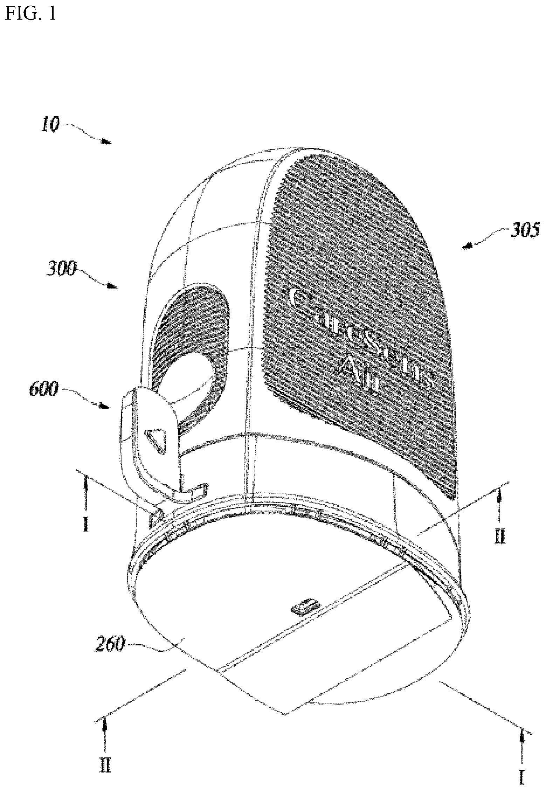

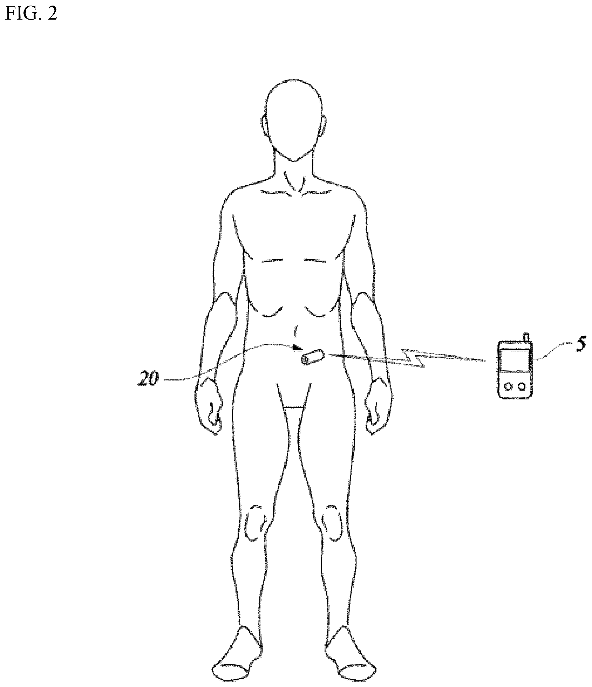

FIG. 1 is a perspective diagram illustrating an applicator assembly according to an example embodiment of the present disclosure. FIG. 2 illustrates a shape in which a medical device of the applicator assembly according to an example embodiment of the present disclosure is attached to skin of a user.

An applicator assembly 10 according to an example embodiment of the present disclosure includes a sensor unit 100 that has a sensor 110 inserted into the skin of the user, a base unit 200 that forms a medical device 20 together with the sensor unit 100, and an applicator 300 for attaching the medical device 20 to the skin of the user. The applicator assembly 10 may be provided to the user in a state in which the sensor unit 100 and the base unit 200 are separately mounted to the applicator 300. The sensor unit 100 and the base unit 200 may be coupled to each other and attached to the skin of the user by the applicator 300.

As illustrated in FIG. 2, the medical device 20 may be attached to the skin of the user to measure biometric information and wirelessly transmit measured data to an external terminal 5. The external terminal 5 may be a portable terminal, an exclusive medical device, a person computer, a sever, or the like that may receive the measured data of the medical device 20. The biometric information which is measurable by the medical device 20 is not particularly limited. As an example embodiment, the medical device 20 may periodically measure blood sugar of the user and transmit blood sugar measurement information to the external terminal 5.

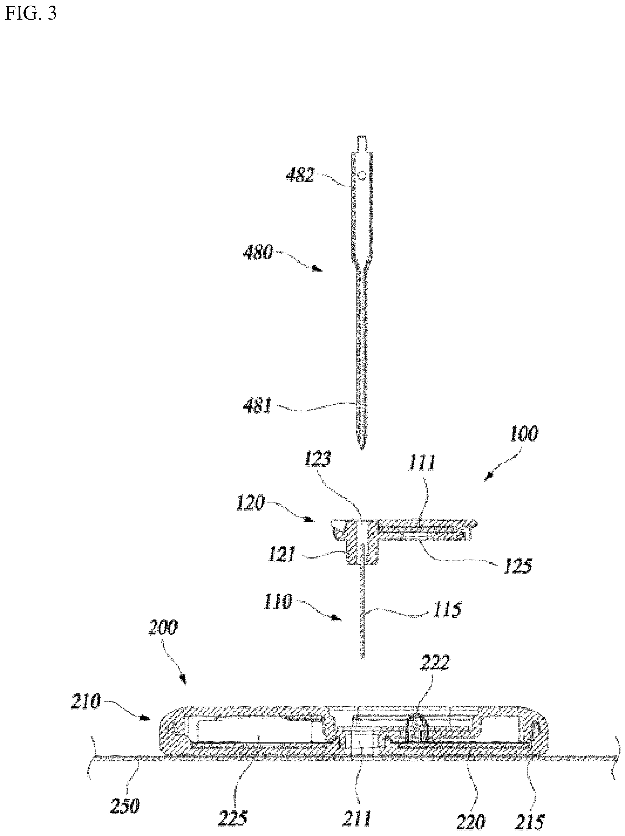

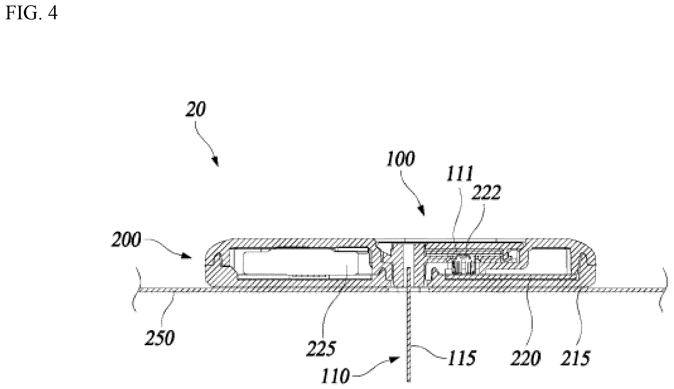

As illustrated in FIGS. 3 and 4, the medical device 20 may include the sensor unit 100 for measuring the biometric information on the user and the base unit 200 which is attached to the skin of the user to be coupled to the sensor unit 100. The base unit 200 may be formed as a configuration in which an electronic component is disposed. The base unit 200 may be electrically connected to the sensor unit 100 to process and transmit biometric information measured by the sensor unit 100 to the external terminal 5.

The sensor unit 100 includes the sensor 110 and a sensor unit housing 120 to which the sensor 110 is coupled. At least a portion of the sensor 110 may be inserted into the skin of the user to detect an analyte in the skin of the user and generate an electrical signal. The sensor 110 includes a sensor body 111 and an insertion part 115 coupled to the sensor 111 to be inserted into the skin of the user. The sensor body 111 is disposed in the sensor unit housing 120. The insertion part 115 protrudes from the sensor unit housing 120.

The sensor unit housing 120 has a boss 121 into which the insertion part 115 of the sensor 110 is inserted. A housing hole 123 that penetrates the sensor unit housing 120 in a thickness-wise direction is formed to the sensor unit housing 120. The insertion part 115 and a needle 480 for inserting the insertion part 115 into the skin of the user are inserted into the housing hole 123. A housing opening 125 is formed to a bottom portion of the sensor unit housing 120. The sensor body 111 may be exposed to an outside of the sensor unit housing 120 through the housing opening 125.

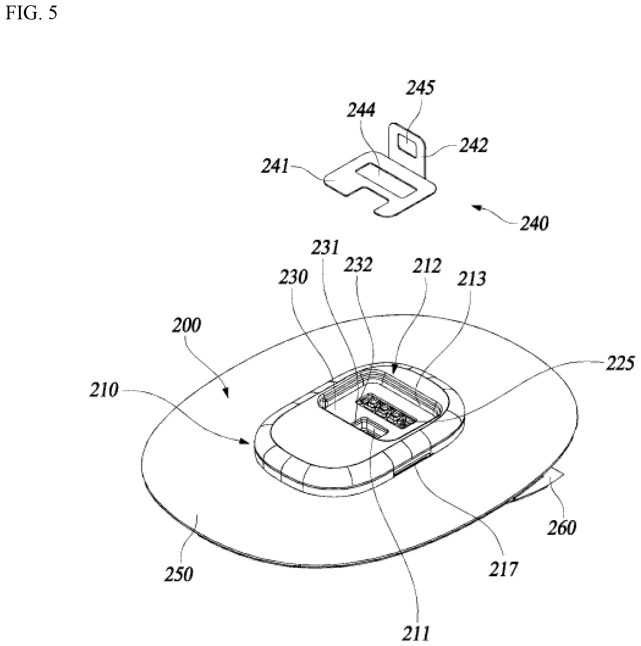

Referring to FIGS. 3 through 5, the base unit 200 includes a base unit housing 210 to which the sensor unit 100 is coupled, an electronic component disposed in the base unit housing 210, and an adhesive layer 230 provided to the base unit housing 210 to be attached to the sensor unit 100. The electronic component may include a substrate 220, an electrical contact part 222 in contact with the sensor 110, a battery 225, and the like. A processor chip for processing a signal, a communication chip for wireless communication with the external terminal 5, or the like may be mounted on the substrate 220.

An insertion hole 211 through which the insertion part 115 of the sensor 110 and the needle 480 may pass and a mounting part 212 to which the sensor unit housing 120 is coupled are provided to the base unit housing 210. The mounting part 212 includes a base unit recess 213. The insertion hole 211 may be formed in a shape to which the boss 121 of the sensor unit 100 is fit and coupled. The insertion hole 211 is disposed in the base unit recess 213 to be extended to a base unit bottom portion 215. The sensor unit housing 120 may be fit and coupled to the base unit recess 213 to maintain a state of stably being coupled to the base unit housing 210. In addition, as the sensor unit housing 120 is stably fit and coupled to the base unit housing 210, moisture or a foreign substance may not easily flow in through a gap between the sensor unit housing 120 and the base unit housing 210. A base unit detent 217 is provided at an outside edge of the base unit housing 210. A locking hook 360 provided to the applicator 300 may engage with the base unit detent 217.

The electrical contact part 222 is in contact with the sensor 110 when the sensor unit 100 is coupled to the base unit 200. The electrical contact part 222 is electrically connected to the substrate 220. When the sensor unit 100 is coupled to the base unit 200, the electrical contact part 222 may pass through the housing opening 125 of the sensor unit housing 120 to be in contact with the sensor body 111. In order to be in stable contact with the sensor body 111, the electrical contact part 222 may be configured to be elastically deformed when being in contact with the sensor body 111.

As the sensor 110 is in contact with the electrical contact part 222, the medical device 20 may be activated. When the medical device 20 is activated, electricity is supplied to the sensor 110 and the electronic component of the base unit 200, and the medical device 20 operates. At this point, the medical device 20 may detect an analyte in body fluids of the user through the sensor 110 and transmit acquired biometric information to the external terminal 5.

As such, in the applicator assembly 10 according to an example embodiment of the present disclosure, the medical device 20 may be activated while the sensor unit 100 and the base unit 200 are coupled. In other words, without additional manipulation for activating the medical device 20, the user may manipulate the applicator 300 to attach the medical device 20 to the skin, so that the medical device 20 may begin to operate. Thus, an additional switch for activating the medical device 20 may not be required, the number of components may be reduced, and a structure of the medical device 20 may be simplified. Also, utilization may be easy because user manipulation for activating the medical device 20 is not required.

The adhesive layer 230 may be disposed on a surface of the base unit housing 210. The adhesive layer 230 may be formed in a shape of double-sided tape of which both sides have adhesiveness. The adhesive layer 230 may attach the sensor unit housing 120 to the base unit housing 210. In a middle portion of the adhesive layer 230, adhesive layer holes 231 and 232 are formed to penetrate the adhesive layer 230 in a thickness-wise direction. One adhesive layer hole 231 is connected to the insertion hole 211. The electrical contact part 222 may protrude above the adhesive layer 230 through another adhesive layer hole 232. The adhesive layer 230 may couple the sensor unit housing 120 and the base unit housing 210 when the sensor unit 100 is coupled to the base unit 200 and may seal the gap between the sensor unit housing 120 and the base unit housing 210 so that the moisture or the foreign substance does not flow in toward the electrical contact part 222.

The adhesive layer 230 is covered with the protective sheet 240 to be protected. The protective sheet 230 is formed of a material separably attached to the adhesive layer 230. When a state in which the adhesive layer 230 is exposed to air is maintained for a long time, adhesiveness of the adhesive layer 230 may be decreased. The protective sheet 240 prevents a decrease in the adhesiveness of the adhesive layer 230 by covering the adhesive layer 230 and allows a worker to easily treat the base unit 200 in a manufacturing process for the base unit 200 or a process of assembling the base unit 200 to the applicator 300. The protective sheet 240 includes a protection part 241 that covers the adhesive layer 230 and a wing part 242 that is extended from the protection part 241. A protective sheet opening 244 is formed to the protection part 241 to penetrate the protection part 241 in a thickness-wise direction. When the protection part 241 covers the adhesive layer 230, the protective sheet opening 244 may be connected to the adhesive layer hole 232 of the adhesive layer 230. A protective sheet hole 245 is formed to the wing part 242 to penetrate the wing part 242 in a thickness-wise direction. The wing part 242 may be extended from the base unit housing 210 toward an outside to be coupled to a moving tab 600 of the applicator 300. A shape of the protective sheet 240 may not be limited to an illustration and may be variously changed.

The base unit 200 is mounted to the applicator 300 in a state in which the protective sheet 240 is attached to the adhesive layer 230. The protective sheet 240 may be separated from the adhesive layer 230 by the moving tab 600 before the sensor unit 100 is coupled to the base unit 200. When the protective sheet 240 is removed from the base unit 200 by the moving tab 600, the sensor unit 100 may be coupled to the base unit 200 by moving toward the base unit 200.

An adhesive pad 250 is provided to the base unit bottom portion 215 of the base unit 200. A size of the adhesive pad 250 is larger than a size of the base unit bottom portion 215. The adhesive pad 250 may attach the base unit housing 210 to the skin of the user. A hole through which the insertion part 115 of the sensor 110 and the needle 480 may pass may be provided in a middle of the adhesive pad 250. The adhesive pad 250 may be covered with a protective sheet 260 to be protected. The protective sheet 260 may be removed in a process of attaching the base unit 200 to the skin of the user.

The sensor unit 100 and the base unit 200 are disposed to the applicator 300 in a mutually separated state. In a process in which the applicator 300 operates to insert the sensor 110 into the skin of the user, the sensor unit 100 and the base unit 200 are coupled to each other to form the medical device 20. The sensor unit 100 may be mounted to the applicator 300 in a state in which the needle 480 is coupled thereto and move toward the base unit 200 to be coupled to the base unit 200 in the state in which the needle 480 is coupled thereto. After the sensor unit 100 is coupled to the base unit 200, the needle 480 is separated from the sensor unit 100, and only the medical device 20 remains on the skin of the user.

As another example embodiment, the base unit 200 may have a configuration of simply supporting the sensor unit 100 without the electronic component so that the sensor unit 100 is not separated from the skin of the user. In this case, an additional electronic unit that may process and transmit the biometric information measured by the sensor unit 100 to the external terminal 5 may be separably coupled to the base unit 200. The additional electronic unit may be coupled to the base unit 200 to be electrically connected to the sensor unit 100 after the sensor unit 100 is coupled to the base unit 200.

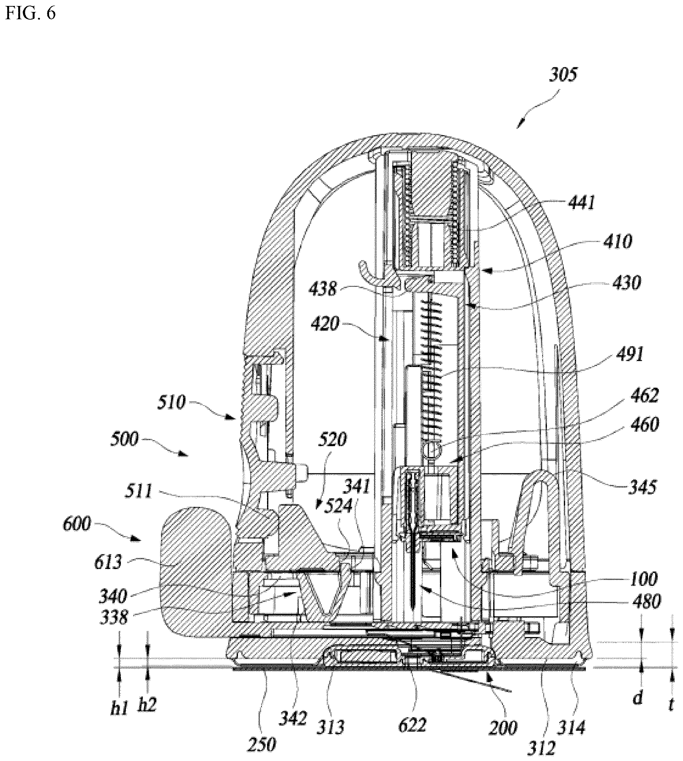

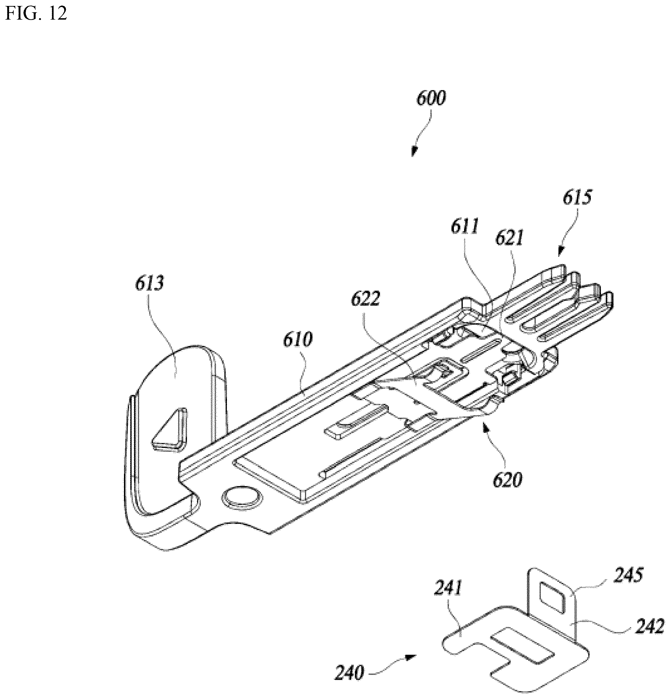

Referring to FIGS. 6 and 12, the applicator 300 may operate, in a state in which the sensor unit 100 and the base unit 200 are mounted thereto, to couple the sensor unit 100 and the base unit 200 and may attach, to the skin of the user, the medical device 20 in which the sensor unit 100 and the base unit 200 are coupled. The applicator 300 is separated from the medical device 20 after the medical device 20 is attached to the skin of the user. The medical device 20 remains in a state of being attached to the skin of the user by the adhesive pad 250.

The applicator 300 includes an applicator body 305 to which the base unit 200 is separably coupled, an insertion unit assembly 400 including an insertion unit 420 for inserting the sensor 110 into the skin of the user, and the moving tab 600 for removing the protective sheet 240. The insertion unit 420 may move the sensor unit 100 from a first position spaced apart from the base unit 200 by a predetermined interval to a second position for being coupled to the base unit 200.

The applicator body 305 includes a base frame 310, a middle frame 330 disposed above the base frame 310, and a top case 350 coupled to the middle frame 330 to cover the middle frame 330.

The base frame 310 has an applicator body bottom portion 312 that may face the skin of the user. An applicator body recess 313 to which the base unit 200 is disposed is provided in a middle of the applicator body bottom portion 312. At least a portion of the base unit 200 may be received to the applicator body recess 313 so that the adhesive pad 250 may faces the skin of the user. A depth d of the applicator body recess 313 is smaller than a thickness t of the base unit 200. Thus, when the base unit 200 is disposed to the applicator body recess 313, the base unit 200 protrudes from the applicator body bottom portion 312. The base unit 200 being coupled to the applicator body 305 to protrude from the applicator body bottom portion 312 is effective in various aspects. That is, as the base unit 200 protrudes from the applicator body bottom portion 312, the user may operate the applicator 300 to insert the sensor 110 into the skin in a state of accurately identifying a position of the base unit 200 on the skin. In addition, when the user grips the applicator 300 and positions the base unit 200 on the skin of the user, the base unit 200 may press and flatten the skin. Thus, the sensor 110 and the needle 480 may be further smoothly inserted into the skin of the user.

A protrusion height h1 to which the base unit 200 protrudes from the applicator body bottom portion 312 is desired to have a size greater than or equal to a half of the thickness t of the base unit 200. When the protrusion height h1 to which the base unit 200 protrudes from the applicator body bottom portion 312 is smaller than the half of the thickness t of the base unit 200, the above described effect is reduced. However, the protrusion height h1 to which the base unit 200 protrudes from the applicator body bottom portion 312 may vary depending on the thickness t of the base unit 200.

A plurality of floor ribs 314 is provided around the applicator body recess 313. The plurality of floor ribs 314 is disposed to be spaced apart around the applicator body recess 313 to protrude from the applicator body bottom portion 312. As being in contact with the adhesive pad 250 of the base unit 200, a floor rib 314 may press the adhesive pad 250 toward the skin of the user. As the floor rib 314 presses the adhesive pad 250 toward the skin of the user in a process of attaching the medical device 20 to the skin of the user, the adhesive pad 250 may be further stably attached to the skin of the user. Also, when the user grips the applicator 300 and positions the base unit 200 on the skin of the user, the plurality of floor ribs 314 may prevent the base unit 200 from being inclined to a side thereof. A drawing illustrates that a protrusion height h2 to which the floor rib 314 protrudes from the applicator body bottom portion 312 is smaller than the protrusion height h1 to which the base unit 200 protrudes from the applicator body bottom portion 312, but a height to which the floor rib 314 protrudes from the applicator body bottom portion 312 may be variously changed.

A base frame opening 315 is formed in a middle of the base frame 310 to penetrate the base frame 310 in a thickness-wise direction. A fence part 316 is provided around the base frame opening 315. The fence part 316 may support the insertion unit assembly 400. A support part 318 is provided at both sides of the base frame opening 315. The support part 318 may tiltably support, the locking hook 360 for separably coupling the base unit 200 to the applicator body 305.

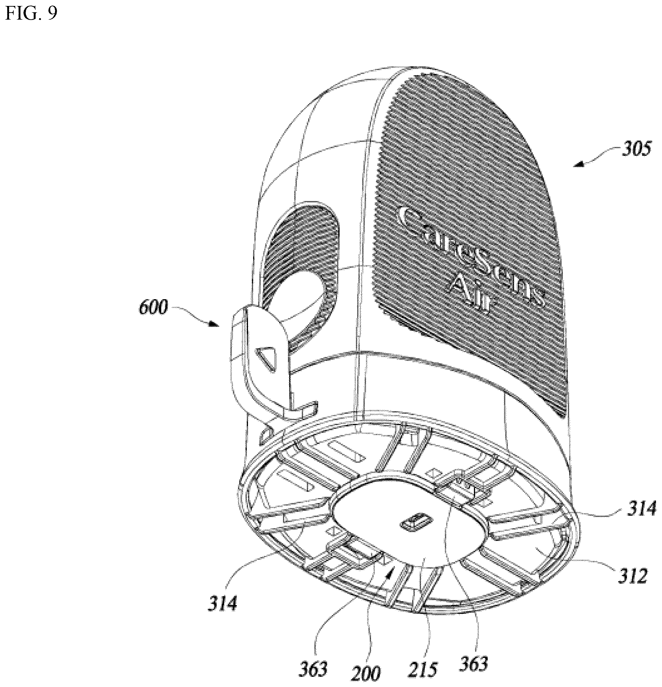

The locking hook 360 may engage with the base unit 200, which is mounted to the applicator body recess 313, to maintain the base unit 200 to be in a state of being disposed to the applicator body recess 313. The locking hook 360 may disengage from the base unit 200 after the medical device 20 is attached to the skin of the user. The locking hook 360 is tiltably coupled to the support part 318. The locking hook 360 includes a shaft 361 rotatably coupled to the support part 318 and a hook part 363 and an extension part 364 that are protruded in a middle of the shaft 361. The hook part 363 protrudes in the middle of the shaft 361. The hook part 363 may protrude from the applicator body bottom portion 312 to engage with a portion of the base unit 200, which protrudes from the applicator body bottom portion 312. As illustrated in FIG. 9, when the base unit 200 is mounted to the applicator body recess 313, the base unit detent 217 of the base unit 200 may be positioned at an outside of the base frame 310, and the hook part 363 may engage with the base unit detent 217 at the outside of the base frame 310.

The movement of the locking hook 360 may be restricted by an operation part 500 in a state in which the hook part 363 engages with the base unit 200, so that the locking hook 360 may maintain the base unit 200 to be in a state of being coupled to the applicator body 300. After the sensor unit 100 is coupled to the base unit 200, restriction of the locking hook 360 by the operation part 500 may be released, so that the locking hook 360 may disengage from the base unit 200.

In addition, a stage 322 and an entrance 323 are provided to the base frame 310. The stage 322 is provided in the base frame 310 to support the moving tab 600 so that the moving tab 600 may linearly move. The entrance 323 is formed at one side of an outer surface of the base frame 310. The moving tab 600 which is disposed to the stage 322 may be withdrawn toward the outside of the base frame 310 through the entrance 323.

A detailed configuration of the base frame 310 may not be limited to an illustration and may be variously changed.

The middle frame 330 includes a stage 331 that supports the operation part 500. A middle frame opening 332 connected to the base frame opening 315 of the base frame 310 is provided in a middle of the stage 331. A column member 410 of the insertion unit assembly 400 may be inserted into the base frame opening 315 through the middle frame opening 332. A guide protrusion 334 is provided at both edges of the stage 331. The guide protrusion 334 may guide the operation part 500 so that the operation part 500 linearly moves from a third position to a fourth position. Here, the third position is a position at which the operation part 500 is unable to operate the insertion unit 420 so that the sensor unit 100 waits at the first position. Also, the fourth position is a position at which the operation part 500 operates the insertion unit 420 so that the sensor unit 100 may move to the second position. When the operation part 500 is moved to the fourth position by the user, the insertion unit 420 may be triggered by the operation part 500 to move the sensor unit 100.

A locking member 338 for restricting movement of the operation part 500 to allow the operation part 500 not to move from the third position and a return member 345 for elastically supporting the operation part 500 are provided to the middle frame 330.

The locking member 338 is disposed in a penetration hole 336 formed in the middle of the stage 331. The locking member 338 includes a body part 340 that is elastically deformably connected to the middle frame 330, a hook part 341 connected to one side of the body part 340 to engage with the operation part 500, and a leg part 342 connected to another side of the body part 340 to be in contact with the moving tab 600. When the leg part 342 is in contact with the moving tab 600, the locking member 338 may slant in a direction in which the hook part 341 engages with the operation part 500. When the leg part 342 is released from the moving tab 600, the locking member 338 may slant in a direction in which the hook part 341 disengages from the operation part 500. In other words, when another external force is not applied, the locking member 338 may maintain a position at which the hook part 341 does not engage with the operation part 500. In addition, when the moving tab 600 is in contact with the leg part 342, the locking member 338 is elastically deformed so that the hook part 341 is in contact with the operation part 500.

A configuration of the locking member 338 may not be limited to an illustration and may be variously changed.

The return member 345 may be in contact with one side of the operation part 500 to elastically support the operation part 500 so that the operation part 500 does not easily move from the third position to the fourth position. That is, the return member 345 may generate a resisting force that resists a force with which the user pushes the operation part 500. As allowing the operation part 500 to move when a pressing force of predetermined magnitude or more is received, the return member 345 may prevent an accidental operation of the operation part 500. Furthermore, the return member 345 provides a moving force that returns, toward the third position, the operation part 500 which has moved to the fourth position. The return member 345 is elastically deformed when the operation part 500 is moved to the fourth position by the user. Also, when a pressing force from the user to the operation part 500 is removed, the return member 345 may move the operation part 500 toward the third position by applying an elastic force to the operation part 500.

A top case 350 covers an upper portion of the middle frame 330. A top case opening 351 to which a button 510 of the operation part 500 is disposed is provided at one side of the top case 350.

In addition to a configuration including the base frame 310, the middle frame 330, and the top case 350 as illustrated, the applicator body 305 may be changed into other various configurations that may be coupled to the insertion unit assembly 400 to support the insertion unit assembly 400 and to which the base unit 200 is separably coupled. Also, the applicator body 305 may be coupled to the base unit 200 with other various schemes in addition to a scheme of using the locking hook 360 of a tilting type.

The insertion unit assembly 400 includes the column member 410 which is fixed to the applicator body 305 and the insertion unit 420 which is supported by the column member 410 to be coupled to the sensor unit 100 and moves the sensor unit 100 from the first position to the second position. The insertion unit 420 includes a shuttle 430 movably disposed in the column member 410 and a needle assembly 450 that may move together with the shuttle 430. The shuttle 430 may be coupled to the sensor unit 100 to move together with the sensor unit 100 from the first position to the second position. The needle assembly 450 includes a carrier 460 that may move relative to the shuttle 430 and the needle 480 which may be coupled to the carrier 460 to be inserted into the skin of the user.

The column member 410 is fixed to the applicator body 305 to support the insertion unit 420. The column member 410 includes a column member body 411 of which a lower end portion is opened outward. A space for receiving the insertion unit 420 is provided in the column member body 411. A release part 414 is provided on an inner surface of the column member body 411. The release part 414 is a portion with which a portion of the carrier 460 is in contact while the carrier 460 moves toward the base unit 200. The carrier 460 may move relative to the shuttle 430 by disengaging from the shuttle 430 due to operation of the release part 414.

The shuttle 430 is linearly movably disposed in the column member 410. The shuttle 430 includes a shuttle body 432 in which a space receiving the needle assembly 450 is provided. A shuttle protrusion 433 is provided at one side of the shuttle body 432. The shuttle protrusion 433 protrudes from the shuttle body 432 to be in contact with the operation part 500. The shuttle 340 is fixed at the first position as the shuttle protrusion 433 is in contact with the operation part 500 and may move to the second position when the shuttle protrusion 433 is released from the operation part 500. A shuttle detent 434 with which a carrier latch 467 of the carrier 460 may engage is provided in the shuttle body 432. In a state in which the carrier latch 467 engages with the shuttle detent 434, the carrier 460 may move together with the shuttle 430 and not move relative to the shuttle 430. Also, when the carrier latch 467 disengages from the shuttle detent 434, the carrier 460 may move relative to the shuttle 430. A shuttle bottom portion 435 in contact with the sensor unit 100 is provided at one end of the shuttle body 432. A shuttle penetration hole 436 is provided to the shuttle bottom portion 435. The needle 480 may protrude toward an outside of the shuttle body 432 through the shuttle penetration hole 436. A fixation part 438 is provided in the shuttle 430. The fixation part 438 is for fixing a needle release driver 490 that provides a moving force to the carrier 460.

The shuttle 430 moves by receiving a moving force from a shuttle driver 440. The shuttle driver 440 includes a forward movement elastic member 441 that applies an elastic force to the shuttle 430 in a direction of movement from the first position to the second position. One end of the forward movement elastic member 441 is contact with one side of the column member 410, and another end thereof is in contact with one side of the shuttle 430. The shuttle 430 may be disposed in the column member 410 in a state in which the forward movement elastic member 441 is elastically deformed. As the shuttle protrusion 433 is in contact with the operation part 500, the shuttle 430 may wait at the first position in the state in which the forward movement elastic member 441 is elastically deformed. In addition, when the operation part 500 moves so that the shuttle protrusion 433 is released from the operation part 500, the shuttle 430 may be moved to the second position by the elastic force of the forward movement elastic member 441.

In addition to a configuration including the forward movement elastic member 441 which has a coil spring shape, the shuttle driver 440 may be changed into other various configurations that may provides a moving force to the shuttle 430.

The needle assembly 450 includes the carrier 460 to which the sensor unit 100 is separably coupled and the needle 480 which is coupled to the carrier 460 to penetrate the sensor unit 100. The needle 480 may be inserted into the skin of the user by moving together with the sensor unit 100 from the first position to the second position in a state of being coupled to the sensor unit 100. Also, the needle 480 may be separated from the skin of the user and the sensor unit 100 by moving backward together with the carrier 460 at the second position.

The carrier 460 is relatively movably coupled to the shuttle 430. As a portion of the carrier 460 engages with the shuttle 430, the carrier 460 may move forward together with the shuttle 430 from the first position to the second position. Furthermore, the carrier 460 may move backward in a direction away from the sensor unit 100 by disengaging from the shuttle 430 at the second position. The carrier 460 includes a carrier body 461 to which the needle 480 is coupled, a plurality of carrier wings 464 extended from the carrier body 461 to be elastically deformed, and a plurality of grip arms 470 connected to a side portion of the carrier body 461. A fixation part 462 to which the needle release driver 490 is coupled is provided to the carrier body 461. A carrier wing 464 includes a wing body 465 that is elastically deformably connected to the carrier body 461, a trigger 466 protruding from the wing body 465, and the carrier latch 467. The trigger 466 may be in contact with the release part 414 of the column member 410 while the carrier 460 moves together with the shuttle 430 from the first position to the second position.

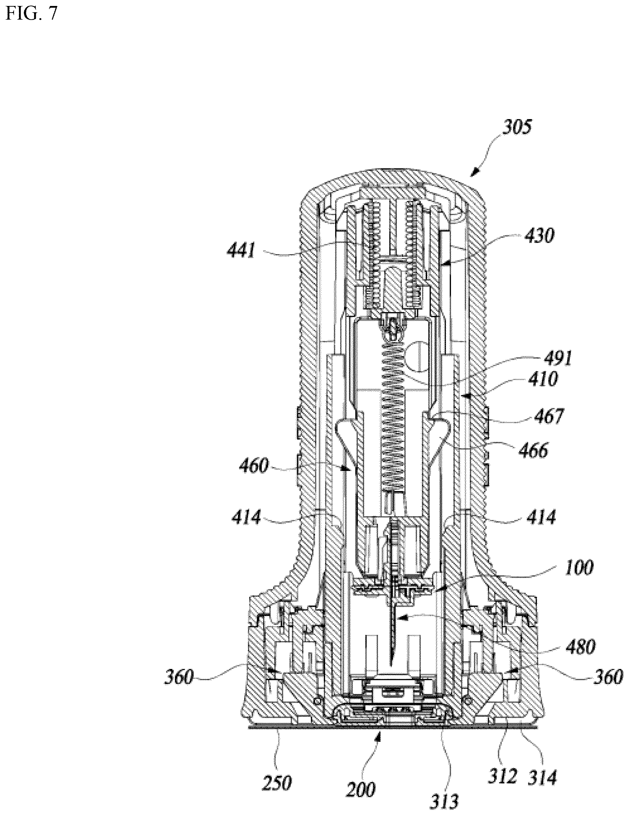

As illustrated in FIG. 7, the release part 414 is provided in the column member 410 to be in contact with the trigger 466. While the carrier 460 moves from the first position to the second position, the trigger 466 may be in contact with the release part 414 to be elastically deformed. The carrier latch 467 protrudes outward from the wing body 465. The carrier latch 467 may engage with the shuttle detent 434 of the shuttle 430. In a state in which the carrier latch 467 engages with the shuttle detent 434, the carrier 460 may maintain a state in which a backward movement elastic member 491 of the needle release driver 490 is elastically deformed and may not move in a direction in which an elastic force of the backward movement elastic member 491 is applied. In addition, when the trigger 466 is in contact with the release part 414 to be elastically deformed, the carrier latch 467 is released from the shuttle detent 434, so that the carrier 460 may move.

A grip arm 470 is elastically deformably provided at the side portion of the carrier body 461. The grip arm 470 may be disposed so that the plurality of the grip arms 470 faces each other at both sides of the carrier body 461 and may engage with the sensor unit 100. The carrier 460 is coupled to the shuttle 430 so that the carrier body 461 is positioned in the shuttle 430 and an end of the grip arm 470 protrudes from the shuttle 430. The carrier 460 may fix the sensor unit 100 with a scheme of the grip arm 470 engaging with the sensor unit housing 120.

Also, the carrier 460 may position, in the applicator body 305, the sensor unit 100 to the first position. When the carrier 460 is positioned at the first position, a portion of the grip arm 470 is in contact with an inner wall 416 of the column member 410. Thus, the grip arm 470 may slant in a direction of engaging with the sensor unit housing 120, and the carrier 460 may maintain a state of being stably coupled to the sensor unit 100.

Meanwhile, when the sensor unit 100 and the carrier 460 move toward the base unit 200 so that the sensor unit 100 is coupled to the base unit 200, the grip arm 470 is released from the inner wall 416 of the column member 410. Thus, a fixing force of the grip arm 470 is weakened in a state in which the sensor unit 100 is coupled to the base unit 200. Since the sensor unit 100 is fixed to the base unit 200 by the adhesive layer 230, when the carrier 460 is pulled in a direction away from the medical device 20 by the needle release driver 490, the grip arm 470 may be separated from the sensor unit 100.

The carrier 460 may be moved relative to the shuttle 430 by the needle release driver 490. The needle release driver 490 includes the backward movement elastic member 491 which has a tension spring shape. The backward movement elastic member 491 may apply the elastic force to the carrier 460 in the direction away from the sensor unit 100 to move the carrier 460 so that the carrier 460 is separated together with the needle 480 from the sensor unit 100. One end of the backward movement elastic member 491 is coupled to the fixation part 438 of the shuttle 430. Also, another end of the backward movement elastic member 491 is coupled to the fixation part 462 of the carrier 460.

The needle release driver 490 may not be limited to an illustration and may be variously changed. As another example embodiment, the needle release driver 490 may include a compression spring that may apply an elastic force to the carrier 460. Furthermore, in addition to a configuration including a spring having a coil spring shape, the needle release driver 490 may be changed into other various configurations that may provides a moving force to the carrier 460.

The needle 480 may move from the first position to the second position in a state of being fixed to the carrier 460 to be coupled to the sensor unit 100. The needle 480 may be formed in a shape with a pointed end to penetrate the skin of the user and be smoothly inserted into the skin of the user. When the sensor unit 100 moves to the second position, the needle 480 may penetrate, earlier than the sensor 110 does, the skin of the user so that the sensor 110 is stably inserted into the skin. After the sensor 110 is inserted into the skin of the user, the needle 480 is separated from the skin of the user. The needle 480 includes a needle body 481 that may be inserted into the skin of the user and a needle head 482 coupled to the carrier 460. A needle coupler 485 for fixing the needle head 482 to the carrier 460 is coupled to the needle head 482. The needle coupler 485 may be fixed to an inside of the carrier 460 to fix the needle head 482 to the carrier 460.

A detailed configuration of the needle 480 may not be limited to an illustration and may be variously changed. In addition, a scheme of coupling the needle 480 and the carrier 460 may be also variously changed.

The insertion unit assembly 400 may be coupled to the applicator body 305 in a state in which the forward movement elastic member 441 of the shuttle driver 440 for moving the shuttle 430 and the backward movement elastic member 491 of the needle release driver 490 for moving the needle assembly 450 are elastically deformed. Also, the insertion unit assembly 400 may be disposed to the applicator body 305 in a state of being coupled to the sensor unit 100. In the applicator assembly 10 according to an example embodiment of the present disclosure, the shuttle 430, the shuttle driver 440, the needle assembly 450, and the needle release driver 490 which form the insertion unit 420 may be simply assembled to the applicator body 305 by forming one assembly together with the column member 410.

The operation part 500 is disposed to the applicator body 305 to be manipulated by the user to move from the third position to the fourth position. The operation part 500 includes the button 510 which may be manipulated by the user and a movement member 520 disposed in the applicator body 305 to be in contact with the shuttle 430. The operation part 500 may be disposed to the applicator body 305 to linearly move in a direction crossing a movement direction of the shuttle 430, further desirably, in a direction perpendicularly crossing the movement direction of the shuttle 430.

The button 510 may be disposed at the top case opening 351 of the applicator body 305 to be pushed by the user. A button protrusion 511 in contact with the movement member 520 is provided at one side of the button 510. When the user pushes the button 510, the button protrusion 511 may push the movement member 520.

The movement member 520 includes a movement member body 521 supported by the stage 331 of the middle frame 330 and a support part 526 for supporting the shuttle 430. A movement member opening 522 is formed to a middle portion of the movement member body 521 to penetrate the movement member body 521 in a thickness-wise direction. A movement member detent 524 is provided at one side of the movement member body 521. The movement member 520 may not move in a state in which the locking member 338 engages with the movement member detent 524. At this point, although the button 510 is manipulated, the movement member 520 may not move from the third position and may maintain a state of supporting the shuttle 430. The movement member 520 may move to the fourth position in a state in which the locking member 338 disengages from the movement member detent 524. In addition, the movement member 520 may be returned toward the third position by an elastic force of the return member 345 after moving to the fourth position.

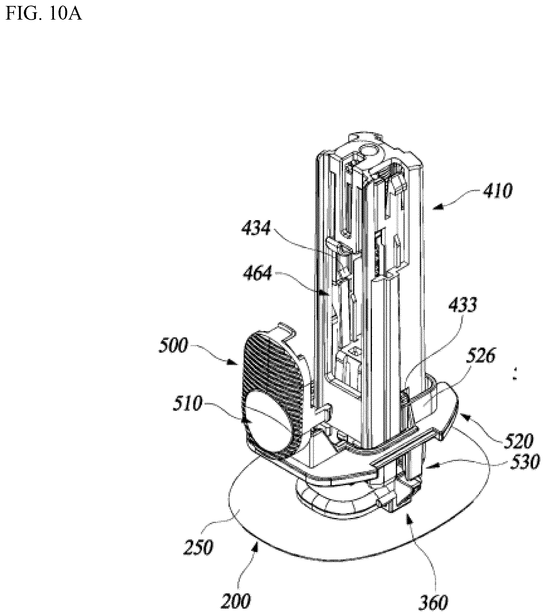

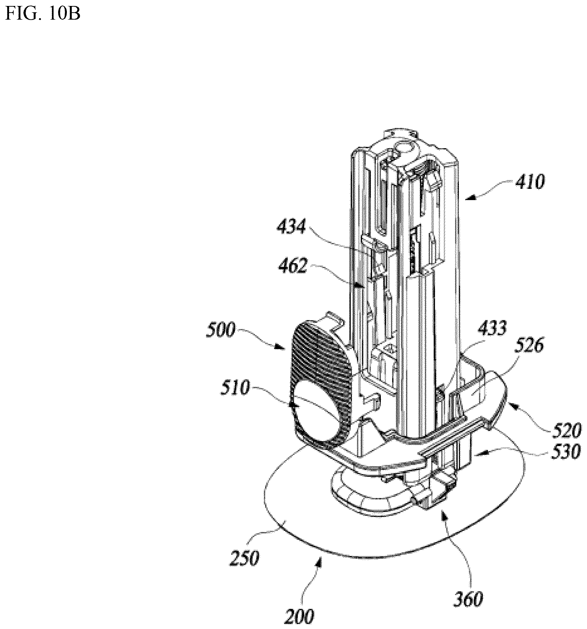

The support part 526 is disposed at an edge of the movement member opening 522. The support part 526 may protrude from the movement member body 521 to support the shuttle protrusion 433 of the shuttle 430. As illustrated in (a) of FIG. 10, when the movement member 520 is positioned at the third position, the support part 526 may support the shuttle protrusion 433 to restrict movement of the shuttle 430 which is disposed at the first position. Also, as illustrated in (b) of FIG. 10, when the movement member 520 moves to the fourth position, as the support part 526 is released from the shuttle protrusion 433, the shuttle 430 may move to the second position.

A stopper 530 that may be in contact with the locking hook 360 is provided to the movement member 520. The stopper 530 protrudes from the movement member body 521 to be in contact with one side of the locking hook 360. When the operation part 500 is positioned at the third position, the stopper 530 may restrict the movement of the locking hook 360 so that the locking hook 360 is not tilted. In contrast, when the operation part 500 is positioned at the fourth position, the stopper 530 may release the restriction of the locking hook 360.

As illustrated in (a) of FIG. 10, when the operation part 500 is positioned at the third position, the stopper 530 is positioned above the extension part 364 of the locking hook 364.

At this point, since the stopper 530 may be in contact with the extension part 364, the locking hook 360 may not be tilted to disengage from the base unit 200. Thus, when the operation part 500 is positioned at the third position, the base unit 200 may be maintained to be coupled to the applicator body 300 in a state in which the locking hook 360 stably engages with the base unit 200. Meanwhile, as illustrated in (b) of FIG. 10, when the operation part 500 is positioned at the fourth position, the stopper 530 moves away from a top of the extension part 364. At this point, the locking hook 360 may be free from interference with the stopper 530 and tilted. Thus, when the operation part 500 is positioned at the fourth position, the locking hook 360 may be tilted to disengage from the base unit 200, and the base unit 200 may be released from the applicator body 300.

The moving tab 600 is manipulated by the user to be coupled to the applicator body 305 so that the protective sheet 240 may be removed. As illustrated in FIG. 12, the moving tab 600 includes a moving tab body 610 connected to the protective sheet 240 and a holder 620 connected to the moving tab body 610. At least a portion of the moving tab 600 may be withdrawn from an inside the applicator body 305 to an outside thereof through an entrance 328 of the applicator body 305. The moving tab 600 may be supported by the stage 322 of the applicator body 305 to linearly move in a direction crossing a movement direction of the sensor unit 100. The moving tab 600 may move from an initial position of being coupled to the protective sheet 240 of the base unit 200 which is positioned at the second position to a removal position at which the protective sheet 240 is separated from the adhesive layer 230.

A moving tab hole 611 penetrating the moving tab body 610 in a thickness-wise direction is formed to the moving tab body 610. The wing part 242 of the protective sheet 240 may be inserted into the moving tab hole 611. A handle part 613 is provided to one end portion of the moving tab body 610, and a moving tab engagement part 615 is provided to another end portion of the moving tab body 610. When the moving tab 600 is positioned at the initial position, the handle part 613 is disposed at an outside of the applicator body 305. When the moving tab 600 is positioned at the initial position, the moving tab engagement part 615 may engage with one inner side of the base frame 310. Thus, when a force of predetermined magnitude or more is applied, the moving tab engagement part 615 disengages from the base frame 310, so that the moving tab 600 may move.

The holder 620 includes a holding part 621 and an elastically deformable pressing part 622. In a state in which the wing part 242 of the protective sheet 240 is inserted into the moving tab hole 611, as the holding part 621 is inserted into the protective sheet hole 245, the protective sheet 240 may be connected to the moving tab 600. The pressing part 622 may protrude from the moving tab body 610 to press the protective sheet 240 which is coupled to the base unit 200. When the moving tab 600 is positioned at the initial position, the pressing part 622 may press the protective sheet 240 toward the adhesive layer 230 to allow protective sheet 240 not to be unattached from the adhesive layer 230. Thus, the protective sheet 240 may maintain a state of stably covering the adhesive layer 230 before the protective sheet 240 is separated from the adhesive layer 230. As being elastically deformable, the pressing part 622 may be in contact with the base frame 310 to be elastically deformed when the moving tab 600 is pulled to the removal position.

The moving tab 600 has a function of restricting, together with the locking member 338, movement of the movement member 520 in addition to a protective sheet removal function. As illustrated in FIG. 6, when the operation part 500 is positioned at the third position, and when the moving tab 600 is positioned at the initial position, the moving tab 600 supports the leg parts 342 of the locking member 338. At this point, the hook part 341 of the locking member 338 maintains a state of engaging with the movement member detent 524 of the operation part 500. Thus, although the operation part 500 is manipulated, the movement member 520 may not move. Meanwhile, when the moving tab 600 is withdrawn from the entrance 323, the locking member 338 may move by disengaging from the operation part 500.

A configuration of the moving tab 600 may not be limited to an illustration and may be variously changed. For example, a drawing illustrates that the handle part 613 protrudes from the applicator body 305 when the moving tab 600 is positioned at the initial position, but the moving tab 600 may have a configuration in which a handling part does not protrudes from the applicator body 305 when the moving tab 600 is positioned at the initial position. Also, when moving to the removal position, the moving tab 600 may be disposed so that only a portion thereof leaves through the entrance 328 and a remaining portion is positioned in the applicator body 305. As another example embodiment, the moving tab may be disposed in a push type. In this case, the moving tab may be disposed to the applicator body 305 to linearly move in the direction crossing the movement direction of the sensor unit 100 and may be moved by the user to remove the protective sheet 240 or remove a restriction force to the operation part 500. Furthermore, the moving tab may be disposed to be movable in various directions of the applicator body 305 to be movable by the user.

In addition, a drawing illustrates that the locking member 338 is disposed to the applicator body 305 to be movable by the moving tab 600, but the moving tab may have a configuration to which the locking member is integrally formed. In this case, the locking member may be provided to the moving tab in an elastically deformable form to engage with the operation part 500 or may be disposed to be elastically supported.

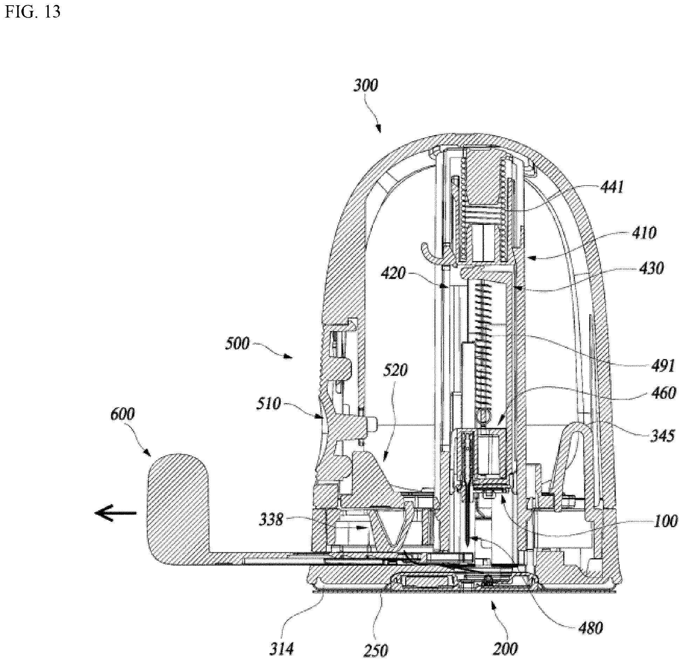

Hereinafter, a process of attaching the medical device 20 to the skin of the user by using the applicator 300 according to an example embodiment of the present disclosure will be described with reference to FIGS. 13 through 16.

To begin with, as illustrated in FIG. 13, the protective sheet 240 is removed by using the moving tab 600. When the moving tab 600 is pulled by the user to move to the removal position, the moving tab 600 may pull the protective sheet 240 in a direction away from the adhesive layer 230 of the base unit 200 to remove the protective sheet 240 from the adhesive layer 230. When the moving tab 600 moves to the removal position, the locking member 338 is released from the moving tab 600. At this point, the locking member 338 disengages from the movement member 520, and the operation part 500 is switched to a movable state by the user.

As such, since the operation part 500 is switched to an operable state when the moving tab 600 moves to the removal position, the applicator 300 may operate after the protective sheet 240 is removed from the base unit 200. Thus, the sensor unit 100 may move toward the base unit 200 after the protective sheet 240 is removed from the base unit 200.

When the insertion unit 420 operates in a state in which the protective sheet 240 is not completely separated so that the sensor unit 100 moves to the second position, the protective sheet 240 may be caught between the sensor unit 100 and the base unit 200, or the sensor unit 100 may be coupled to the base unit 200 in the state in which the protective sheet 240 is not completely separated. As allowing a separation operation of the protective sheet 240 to be completed before the sensor unit 100 moves from the first position, the applicator 300 according to the present example embodiment may prevent the sensor unit 100 from reaching the base unit 200 in a state in which the protective sheet 240 is not separated. Thus, a danger of a malfunction due to carelessness of the user may be reduced.

The applicator 300 may operate in a state in which the adhesive pad 250 of the base unit 200 is in contact with the skin of the user and move the sensor unit 100 toward the skin of the user. Since the base unit 200 protrudes from the applicator body bottom portion 312 of the applicator body 305, the user may operate the applicator 300 while identifying the position of the base unit 200 which is disposed on the skin. When the user grips the applicator 300 and positions the base unit 200 on the skin, as the base unit 200 presses the skin of the user, the skin on which the base unit 200 is disposed may be flattened. Also, the plurality of floor ribs 314 which is provided to the applicator body 305 may press and stably attach the adhesive pad 250 to the skin.

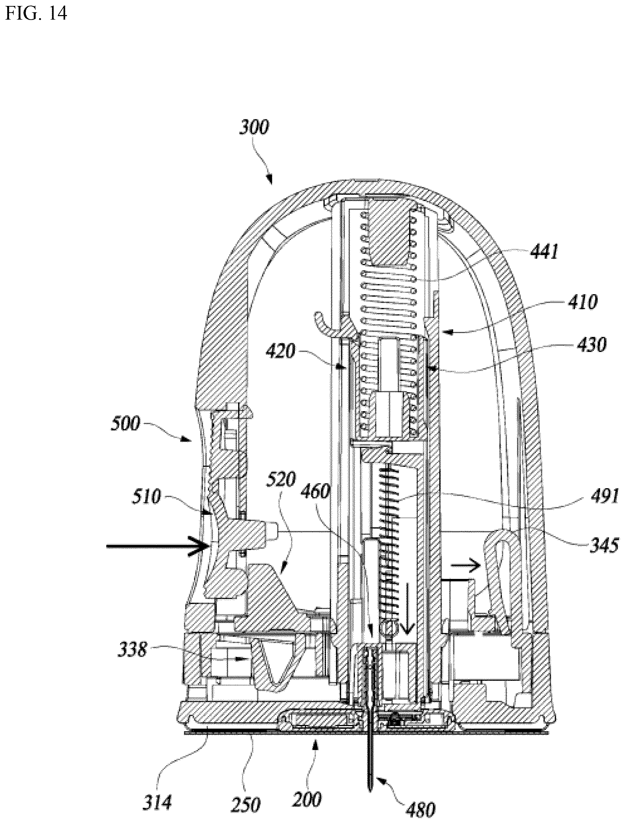

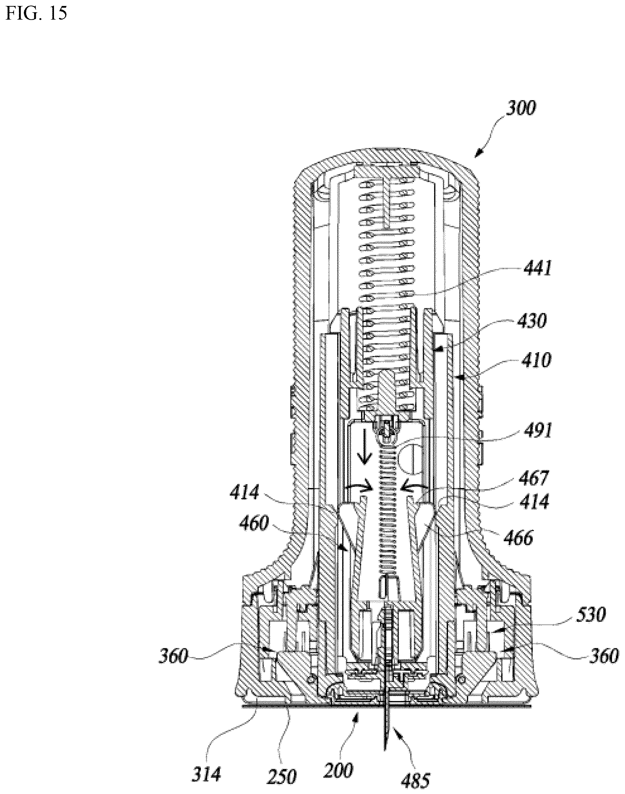

As illustrated in FIGS. 14 and 15, when the operation part 500 is pushed by the user, the operation part 500 moves to the fourth position while elastically deforming the return member 345. As the operation part 500 moves to the fourth position, the shuttle 430 is released from the movement member 520 and moved to the second position by the forward movement elastic member 441. As the shuttle 430 moves to the second position, the needle 480 and the sensor 110 are inserted into the skin of the user, and the sensor unit 100 is attached to the base unit 200 by the adhesive layer 230. When the shuttle 430 moves to the second position, the trigger 466 of the carrier 460 is in contact with the release part 414 of the column member 410. At this point, as the carrier wing 464 of the carrier 460 is elastically deformed, the carrier latch 467 disengages from the shuttle detent 434.

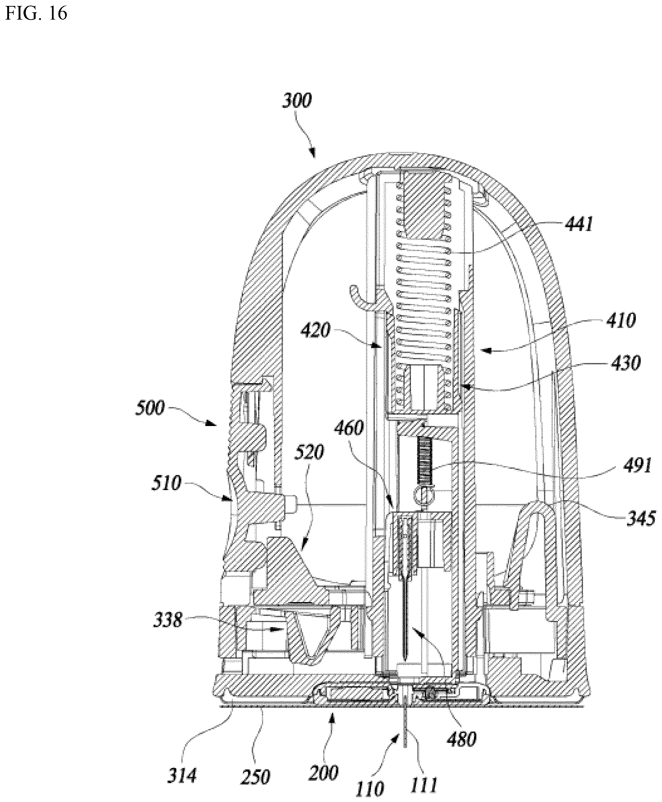

When the carrier 460 disengages from the shuttle 430, as illustrated in FIG. 16, the carrier 460 is moved by the backward movement elastic member 491 in a direction away from the skin of the user. At this point, the needle 480 leaves the skin of the user, and the medical device 20 maintains a state of being attached to the skin of the user by the adhesive pad 250.

After the sensor 110 is inserted into the skin of the user, the applicator body 300 may be separated from the medical device 20 which is attached to the skin. In other words, when the user lifts the applicator 300 in the direction away from the skin of the user, the locking hook 360 may be tilted to disengage from the medical device 20, and the applicator 300 may be separated from the medical device 20.

As described above, in the applicator assembly 10 according to an example embodiment of the present disclosure, since the base unit 200 is mounted to the applicator body 305 to protrude from the applicator body bottom portion 312, the user may insert the sensor 110 into the skin while identifying the position of the base unit 200 which is disposed on the skin.

Also, in the applicator assembly 10 according to an example embodiment, the base unit 200 which protrudes from the applicator body bottom portion 312 may press and flatten the skin into which the sensor 110 is to be inserted. Thus, the sensor 110 and the needle 480 may be further stably inserted into the skin.

The present disclosure has been described above with desired examples. However, the scope of the present disclosure is not limited to the form described and illustrated above.

For example, a drawing illustrates that the sensor unit is separably coupled to the carrier coupled to the needle and moves from the first position to the second position, but the sensor unit may be separably coupled to the shuttle or an additional member of the shuttle to move together with the shuttle.

In addition, a drawing illustrates that the button of the operation part and the movement member are formed as individually different components, but the button and the movement member may be integrally formed.

Also, a drawing illustrates that the protective sheet coupled to the base unit may be separated by the moving tab linearly movably coupled to the applicator body, but the protective sheet may be removed with other various schemes.

In addition, the sensor unit and the base unit may be coupled with other various schemes in addition to a scheme of using the adhesive layer.

Furthermore, a drawing illustrates that the applicator body recess which is provided to receive the base unit is formed in a shape corresponding to a portion of an upper portion of the base unit to receive the base unit partially, but a shape or a size of the applicator body recess may be variously changed.

Being illustrated as being provided, but the applicator may not include the applicator body recess. In this case, the locking hook of the applicator may support the base unit so that the base unit is positioned at a side of the applicator body bottom portion.

As described above, the present disclosure is illustrated and described with desirable example embodiments for show an example of the principle of the present disclosure. However, the present disclosure is not limited to configurations and actions illustrated and described therein. Instead, those skilled in the art may easily understand that multiple changes and corrections in the present disclosure are allowed without deviating from the spirit and the scope of the accompanying claims.

## Claims

1. An applicator assembly comprising:
a medical device comprising a sensor unit having a sensor of which at least a portion is to be inserted into skin of a user and a base unit attached to the skin of the user to be coupled to the sensor unit;
an applicator body comprising an applicator body bottom portion disposed to face the skin of the user and an applicator body recess formed to the applicator body bottom portion wherein the base unit is disposed; and
an insertion unit disposed to the applicator body for moving the sensor unit from a first position spaced apart from the base unit to a second position for being coupled to the base unit,
wherein the base unit is disposed to the applicator body recess to protrude from the applicator body bottom portion.

2. The applicator assembly of claim 1, wherein the applicator body comprises a plurality of floor ribs disposed around the applicator body recess to protrude from the applicator body bottom portion.

3. The applicator assembly of claim 1, comprising a locking hook that is provided on the applicator body, wherein at least a portion of the locking hook protrudes from the applicator body bottom portion and that is configured to engage with a portion of the base unit, wherein the portion of the base unit protrudes from the applicator body bottom portion.

4. The applicator assembly of claim 1, wherein the sensor is configured to be in contact with an electrical contact part of the base unit when the sensor unit moves to the second position to be coupled to the base unit, and
the medical device is configured to be activated as the sensor is in contact with the electrical contact part.

5. The applicator assembly of claim 1, wherein a protrusion height to which the base unit protrudes from the applicator body bottom portion has a size greater than or equal to a half of a thickness of the base unit.

6. An applicator for attaching, to skin of a user, a medical device comprising a sensor unit having a sensor of which at least a portion is to be inserted into the skin of the user and a base unit attached to the skin of the user to be coupled to the sensor unit, the applicator comprising:
an applicator body comprising an applicator body bottom portion disposed to face the skin of the user and an applicator body recess formed to the applicator body bottom portion wherein the base unit is disposed; and
an insertion unit disposed to the applicator body for moving the sensor unit from a first position spaced apart from the base unit to a second position for being coupled to the base unit,
wherein a depth of the applicator body recess is smaller than a thickness of the base unit wherein the base unit is to protrudes from the applicator body bottom portion when the base unit is disposed to the applicator body recess.
